# FASCICULE DE BREVET EUROPEEN

(11) **EP 0 967 941 B1**
(45) Date de publication et mention de la délivrance du brevet: **18.09.2002**
(21) Numéro de dépôt: 99900495.5
(22) Date de dépôt: 05.01.1999
(51) Int. Cl.: A61F 2/40, A61F 2/46

(54) **EQUIPEMENT CHIRURGICAL POUR L'IMPLANTATION D'UNE PROTHESE TOTALE D'EPAULE, ET PROTHESE TOTALE D'EPAULE CONSTITUTIVE**
CHIRURGISCHE AUSRÜSTUNG ZUR IMPLANTATION EINER SCHULTER-TOTALPROTHESE, UND DEMENTSPRECHENDE SCHULTER-TOTALPROTHESE
SURGICAL EQUIPMENT FOR IMPLANTING A TOTAL SHOULDER PROSTHESIS, AND TOTAL SHOULDER PROSTHESIS CONSTITUTING SAME

(30) Priorité: 09.01.1998 FR 9800146
(43) Date de publication de la demande: 05.01.2000
(73) Titulaire: Léonard, Alain, 65500 Caixon (FR); Flurin, Pierre, 33000 Bordeaux (FR)
(72) Inventeur: Léonard, Alain, 65500 Caixon (FR); Flurin, Pierre, 33000 Bordeaux (FR)
(74) Mandataire: Cabinet BARRE LAFORGUE & associés
(86) Numéro de dépôt international: FR9900007
(87) Numéro de publication internationale: WO99034756

(56) Documents cités:
- EP-A- 0 549 480
- EP-A- 0 679 375
- EP-A- 0 712 617
- WO-A-97/25943
- FR-A- 2 664 809
- FR-A- 2 727 857
- FR-A- 2 731 612
- FR-A- 2 742 038
- US-A- 5 064 427

## Description

L'invention concerne un équipement chirurgical pour l'implantation d'une prothèse totale d'épaule. Elle s'étend à une prothèse totale d'épaule du type comprenant une queue de prothèse destinée à être implantée dans le canal huméral d'un patient et une calotte dite sphérique destinée à coopérer avec la glène prothétique ou non de l'épaule.

Initialement, les premières prothèses d'épaule dites de NEER ont consisté en des prothèses monobloc, c'est-à-dire comportant une queue de prothèse et une calotte sphérique d'un seul tenant. Toutefois, il s'est avéré à l'usage que de telles prothèses présentent deux inconvénients majeurs.

En effet, et en premier lieu, le caractère monobloc de ces prothèses impose de constituer un stock important d'implants différents en vue de pouvoir s'adapter aux exigences anatomiques des divers patients. De plus, la position relative de la calotte sphérique et de la queue de prothèse étant figée, du fait également du caractère monobloc de la prothèse, la seule solution offerte au praticien pour reproduire exactement l'anatomie de l'extrémité supérieure de l'humérus, consiste à adapter l'humérus à ladite prothèse, opération qui s'avère très délicate et source d'échecs ou tout le moins de traumatismes ultérieurs pour le patient.

Pour pallier ces inconvénients, plusieurs solutions ont été proposées, décrites notamment dans les brevets français FR-2 685 633, FR-2 727 002, FR-2 727 857 et FR-2 731 612, visant à fournir des prothèses comportant des calottes et des queues de prothèses associées par l'intermédiaire de moyens de liaison aptes à permettre d'ajuster leur positionnement relatif par rotation de la calotte autour de deux ou trois axes de rotation par rapport à la queue de prothèse, puis de bloquer ces éléments dans la configuration souhaitée.

De telles prothèses présentent comme avantage, par rapport aux prothèses NEER, de permettre de se rapprocher de l'anatomie exacte de l'extrémité supérieure de l'humérus. Toutefois, elles présentent également deux inconvénients majeurs.

En effet, et en premier lieu, de par la conception des systèmes de blocage relatif de la calotte et de la queue de prothèse, elles imposent, dans un premier temps, d'utiliser des prothèses d'essai permettant au praticien de déterminer le positionnement idéal relatif de la calotte et de la queue de prothèse, puis dans un second temps, de dupliquer la géométrie de la prothèse d'essai de façon à réaliser la prothèse définitive destinée à être implantée.

Or, ce mode opératoire conduit à augmenter la durée de l'intervention au cours de laquelle le patient est maintenu sous anesthésie. De plus, il peut être la sources d'erreurs préjudiciables pour ce patient lors de la duplication de la prothèse d'essai qui doit être effectuée en cours d'intervention, c'est-à-dire dans un temps le plus réduit possible. Il est à noter, en outre, que compte tenu de l'obligation de dupliquer la prothèse d'essai, toute augmentation des possibilités de réglage permettant avantageusement d'améliorer la reproduction de l'anatomie de l'extrémité supérieure de l'humérus, s'avère conduire à une complication de l'étape de duplication de cette prothèse d'essai. Ainsi, notamment, il apparaît que cette étape de duplication s'avère des plus délicates pour les prothèses décrites dans les brevets FR-2 727 002 et FR-2 727 857 qui offrent la gamme de réglages la plus performante.

Le second inconvénient de ces prothèses résulte du fait que des études anatomiques ont permis de révéler l'existence d'une double excentration, dite déport combiné, entre l'axe métaphysaire proximal de l'humérus et l'axe parallèle passant par le centre de la tête humérale. Ce déport combiné présente une composante médiale relativement constante d'un individu à un autre, dite déport médial, et une composante postérieure, dite déport postérieur, relativement variable d'un individu à un autre, entre lesquelles n'existe aucune relation. Or, aucune des prothèses actuelles telles que notamment décrites dans les brevets français précités, ne permet de régler de façon indépendante le déport médial et le déport postérieur, de sorte qu'une reproduction exacte de l'extrémité supérieure de l'humérus ne peut, à ce jour, être obtenue. De plus, cette impossibilité de réglage indépendant des deux déports peut conduire le praticien à augmenter artificiellement la rétrotorsion humérale lors de la pose.

Le document FR-A-2727002 réfléchit l'état de la technique le plus proche. Le document décrit une prothèse totale d'épaule qui comprend:
- une queue de prothèse d'épaule comportant une tige humérale, et une partie métaphysaire coudée par rapport à ladite tige humérale, dotée d'une face frontale supérieure inclinée par rapport à la tige humérale dans laquelle est ménagée une cavité hémisphérique axée sur un axe (α), et d'un alésage débouchant dans le fond de la cavité hémisphérique et axé sur l'axe (α),
- une platine humérale comportant :
   . une rotule hémisphérique d'axe de révolution D, présentant une face externe adaptée pour coopérer avec la cavité de la queue de prothèse, percée axialement d'un alésage ,
   . un pivot tronconique d'axe de révolution D' relié à la rotule par un tronçon de jonction de section au plus égale à la section maximale de ladite rotule, lesdits pivot conique et tronçon de jonction étant percés d'un alésage traversant s'étendant dans le prolongement de la cavité de la rotule,
- un organe de blocage comportant:
   . une tige fileté
   . et une tête de vissage dudit organe de blocage, et:
- une calotte comportant une paroi supérieure concave destinée à coopérer avec la glène de l'épaule et une paroi inférieure présentant un évidement tronconique de forme conjuguée du pivot tronconique.

La présente invention vise à pallier les inconvénients des prothèses d'épaule actuelles et a pour premier objectif de fournir une prothèse d'épaules offrant une pluralité de réglages continus de la position de la calotte relativement à la queue de prothèse, et dont ladite queue de prothèse peut être scellée directement sans faire appel à des prothèses d'essai.

Un autre objectif de l'invention est de fournir une prothèse d'épaule autorisant des réglages continus indépendants du déport médial et du déport postérieur.

A cet effet, l'invention vise, en premier lieu, un équipement chirurgical comprenant
- une queue de prothèse d'épaule comportant une tige humérale, et une partie métaphysaire coudée par rapport à ladite tige humérale, dotée d'une face frontale supérieure inclinée par rapport à la tige humérale dans laquelle est ménagée une cavité hémisphérique axée sur un axe (α), et d'un alésage taraudé débouchant dans le fond de la cavité hémisphérique et axé sur l'axe (α),
- une platine humérale comportant :
   . une rotule hémisphérique creuse d'axe de révolution D, présentant une face externe adaptée pour coopérer avec la cavité de la queue de prothèse et une face interne définissant une portée hémisphérique, et étant percée axialement d'un alésage tronconique,
   . un pivot tronconique d'axe de révolution D' excentré d'une distance d par rapport à l'axe D, relié à la rotule par un tronçon de jonction de section au plus égale à la section maximale de ladite rotule, lesdits pivot conique et tronçon de jonction étant percés d'un alésage traversant s'étendant dans le prolongement de la cavité de la rotule,
- un organe de blocage comportant disposés axialement:
   . une tige dotée d'un tronçon inférieur fileté, et présentant un diamètre conjugué de celui de l'alésage de la queue de prothèse et inférieur au diamètre minimal de l'alésage tronconique de la rotule de la platine humérale,
   . une rotule hémisphérique adaptée pour coopérer avec la partie hémisphérique de la rotule de la platine humérale,
   . et une tête de vissage dudit organe de blocage.
- les cavités hémisphériques de la queue de prothèse, les faces externe et interne de la rotule de la platine humérale et la rotule de l'organe de blocage étant concentriques autour d'un point O en position verrouillée de l'organe de blocage,
- une calotte comportant une paroi supérieure concave destinée à coopérer avec la glène de l'épaule et une paroi inférieure présentant un évidement tronconique de forme conjuguée du pivot tronconique,
- une calotte, dite d'essai, comportant une paroi inférieure de mêmes dimensions que celles de la paroi inférieure de la calotte présentant un évidement tronconique de forme conjuguée du pivot tronconique, et une paroi supérieure percée d'une ouverture débouchant dans ledit évidement.

(Il est à noter que par rotules et portées hémisphériques, on entend définir des éléments de forme générale hémisphérique définissant un arc de cercle égal ou sensiblement supérieur à 180 degrés.)

Selon l'invention, l'équipement chirurgical comporte, en premier lieu, une queue de prothèse d'épaule dont les éléments constitutifs, tige humérale, platine humérale, et organe de blocage relatif desdites tige humérale et platine humérale, présentent des portées de contact hémisphériques, et sont conçus et agencés de façon que les rotules de la platine humérale et de l'organe de blocage puissent osciller dans toutes les directions autour d'un point 0 constituant le centre de la cavité hémisphérique de la tige humérale. Cet équipement chirurgical comporte, en outre, des calottes d'essai présentant une paroi inférieure de mêmes dimensions que celle des calottes définitives, et adaptées pour permettre, d'une part de régler la position de la platine humérale, et d'autre part de verrouiller l'organe de blocage et donc de bloquer en position souhaitée ladite platine humérale par rapport à la tige humérale.

Un tel équipement chirurgical a pour premier avantage de permettre de sceller directement la queue de prothèse définitive, et d'effectuer les réglages de la position de la calotte, moyennant l'utilisation de calottes d'essai spécifiques, une fois ce scellement réalisé. En effet, il ne nécessite donc pas ainsi d'utiliser des prothèses d'essai destinées à être dupliquées, et conduit par là-même à une simplification et à une réduction du temps de l'intervention chirurgicale.

De plus, cet équipement chirurgical autorise d'effectuer, en continu, le réglage de l'inclinaison de la calotte sphérique, le réglage de la rétroversion, et le réglage du déport combiné. En effet :
- les réglages de la rétroversion et de l'inclinaison sont obtenus par le biais de l'articulation que constituent la cavité hémisphérique de la queue de prothèse et la rotule de la platine humérale, et qui autorise des variations d'orientation de ladite platine humérale centrées sur le point 0,
- les réglages du déport combiné sont obtenus grâce à l'excentricité d entre l'axe de révolution D de la rotule de la platine humérale et l'axe de révolution D' du pivot de ladite platine humérale.

Selon une autre caractéristique de l'invention, les calottes présentent un axe de révolution D" excentré d'une distance d' par rapport à l'axe de révolution D' du pivot tronconique de la platine humérale.

Cette disposition permet de régler de façon indépendante les notions de déport postérieur et de déport médial, et conduit ainsi à l'obtention d'un recouvrement optimal de la coupe osseuse par la calotte sphérique.

En effet, les excentricités d, d' permettent de faire décrire à l'axe D" de la calotte d'essai un mouvement de satellite par rapport à l'axe de la tige humérale, et d'obtenir un réglage fin, continu et surtout indépendant des notions de déports médial et postérieur.

Selon une caractéristique complémentaire de l'invention, l'équipement chirurgical comprend un préhenseur doté d'un manche et d'une tête de préhension inclinée par rapport audit manche, ladite tête de préhension étant adaptée pour obturer la cavité de la partie métaphysaire de la queue de prothèse et comportant :
- un organe de blocage doté d'une tige filetée apte à être vissée dans l'alésage taraudé de la partie métaphysaire de la queue de prothèse,
- une couronne montée autour de ladite tête de préhension avec une faculté d'oscillation autour d'un point O' de l'axe (α) situé à une distance du fond de la cavité de la queue de prothèse inférieure à celle séparant le point O dudit fond de cavité.

Un tel préhenseur est adapté pour être assemblé de façon rigide, par l'intermédiaire de l'organe de blocage, avec la queue de prothèse dépourvue de la platine humérale, de façon à permettre l'introduction de cette dernière dans le canal huméral.

En outre, ce préhenseur est conçu, notamment grâce à la position du point O' autour duquel oscille la couronne, décalé par rapport au point O, pour permettre d'implanter la queue de prothèse de façon que la partie métaphysaire de cette dernière soit positionnée de façon optimale, garantissant la possibilité ultérieure d'effectuer les réglages autorisés par les liaisons rotulées de ladite prothèse.

Enfin, la tête de préhension de ce préhenseur obturant la cavité hémisphérique de la queue de prothèse, garantit contre toute introduction de ciment à l'intérieur de cette cavité lors du scellement de ladite queue de prothèse.

Selon une autre caractéristique complémentaire de l'invention, la tête de préhension du préhenseur comporte :
- un capuchon creux de forme adaptée pour coiffer la face frontale de la queue de prothèse, présentant une cavité interne formant une portée hémisphérique, et comprenant une paroi supérieure percée axialement d'un orifice, et une paroi latérale percée d'orifices oblongs longitudinaux,
- une rotule logée dans la cavité interne du capuchon et percée axialement d'un alésage de diamètre supérieur à celui de l'alésage taraudé de la queue de prothèse,
- et des éléments de liaison de la rotule et de la couronne agencés pour s'étendre au travers des orifices oblongs du capuchon et pour autoriser les débattements angulaires desdites rotule et couronne,
- l'organe de blocage consistant en une vis dotée d'une tige agencée pour s'étendre au travers de l'orifice du capuchon et de l'alésage de la rotule.

L'équipement chirurgical selon l'invention comprend, en outre, avantageusement un jeu de platines humérales présentant des tronçons de jonction de hauteurs différentes.

Ce jeu de platines humérales offre, en effet, la possibilité d'effectuer un réglage en translation afin de restituer éventuellement la longueur normale de l'humérus, réglage qui peut, en outre, être affiné en prévoyant classiquement un jeu de calottes d'épaisseurs différentes.

Selon une autre caractéristique complémentaire de l'invention, les calottes d'essai comportent une tête de préhension moletée délimitant l'évidement tronconique et dotée d'une paroi supérieure ouverte, et une couronne circulaire externe s'étendant sur le pourtour de la base de ladite tête moletée.

L'invention s'étend à une prothèse d'épaule comprenant :
- une queue de prothèse d'épaule comportant une tige humérale, et une partie métaphysaire coudée par rapport à ladite tige humérale, dotée d'une face frontale supérieure inclinée par rapport à la tige humérale dans laquelle est ménagée une cavité hémisphérique axée sur un axe (α), et d'un alésage taraudé débouchant dans le fond de la cavité hémisphérique et axé sur l'axe (α),
- une platine humérale comportant :
   . une rotule hémisphérique creuse d'axe de révolution D, présentant une face externe adaptée pour coopérer avec la cavité de la queue de prothèse et une face interne définissant une portée hémisphérique, et étant percée axialement d'un alésage tronconique,
   . un pivot tronconique d'axe de révolution D' excentré d'une distance d par rapport à l'axe D, relié à la rotule par un tronçon de jonction de section au plus égale à la section maximale de ladite rotule, lesdits pivot conique et tronçon de jonction étant percés d'un alésage traversant s'étendant dans le prolongement de la cavité de la rotule,
- un organe de blocage comportant disposés axialement :
   . une tige dotée d'un tronçon inférieur fileté, et présentant un diamètre conjugué de celui de l'alésage de la queue de prothèse et inférieur au diamétral minimal de l'alésage tronconique de la rotule de la platine humérale,
   . une rotule hémisphérique adaptée pour coopérer avec la partie hémisphérique de la rotule de la platine humérale,
   . et une tête de vissage dudit organe de blocage.
- les cavités hémisphériques de la queue de prothèse, les faces externe et interne de la rotule de la platine humérale et la rotule de l'organe de blocage étant concentriques autour d'un point O en position verrouillée de l'organe de blocage,
- une calotte comportant une paroi supérieure concave destinée à coopérer avec la glène de l'épaule et une paroi inférieure présentant un évidement tronconique de forme conjuguée du pivot tronconique.

De plus, de façon avantageuse, la calotte présente un axe de révolution D" excentré d'une distance d' par rapport à l'axe de révolution D' du pivot tronconique de la platine humérale.

D'autres caractéristiques, buts et avantages de l'invention ressortiront de la description détaillée qui suit en référence aux dessins annexés qui en représentent, à titre d'exemple non limitatif, un mode de réalisation préférentiel. Sur ces dessins qui font partie intégrante de la présente description :
- la figure 1 est une vue en perspective en mode éclaté des divers éléments constitutifs d'une prothèse humérale conforme à l'invention,
- la figure 2 est une coupe longitudinale axiale en mode éclaté de ces éléments, la queue de prothèse étant représentée partiellement en vue latérale,
- la figure 3 est une coupe longitudinale axiale de cette prothèse humérale, dans le mode assemblé des divers éléments constitutifs,
- la figure 4 est une vue en perspective partielle, vue de dessus, d'une prothèse humérale selon l'invention équipée d'une calotte d'essai,
- la figure 5 est une coupe longitudinale axiale de la prothèse humérale de la figure 4,
- la figure 6 est une vue en perspective d'un préhenseur destiné à implanter une queue de prothèse humérale selon l'invention,
- la figure 7 est une vue en perspective partielle, en mode éclaté, des divers éléments constitutifs de ce préhenseur,
- la figure 8 est une vue en perspective partielle de ce préhenseur et d'une queue de prothèse humérale, dissociés,
- et la figure 9 est une coupe longitudinale axiale d'un préhenseur et d'une queue de prothèse humérale assemblés.

L'équipement chirurgical conforme à l'invention représenté aux figures comprend principalement une prothèse humérale complète, un préhenseur destiné à permettre d'implanter la queue de prothèse humérale à l'intérieur d'un canal huméral, et des calottes dites d'essai permettant de réaliser le réglage des paramètres de reconstitution de l'extrémité supérieure de l'humérus.

De façon classique, il est bien entendu que cet équipement chirurgical comporte un jeu de queues de prothèses de longueurs et de diamètres différents. De même, il comprend un jeu de calottes de diamètres et de hauteur différentes ainsi qu'un jeu de calottes d'essai de même diamètre que lesdites calottes.

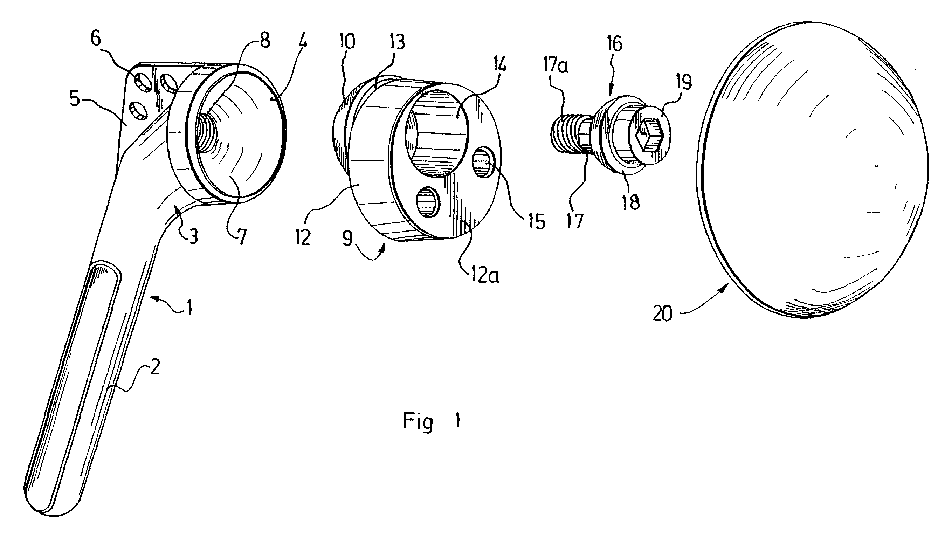

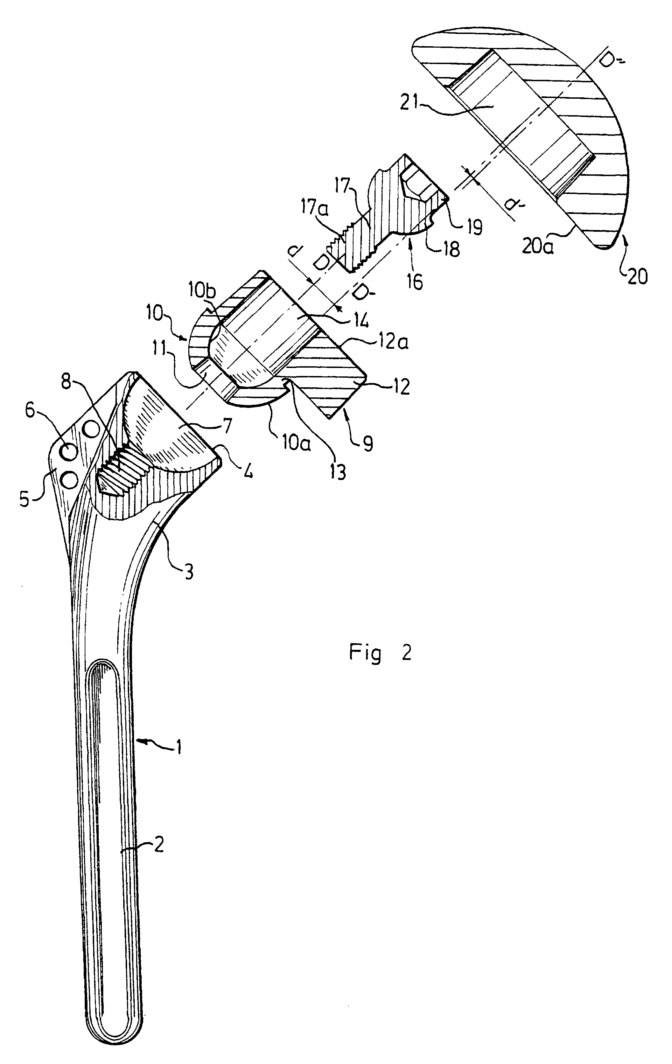

La prothèse humérale conforme à l'invention représentée aux figures 1 à 3 comporte, en premier lieu, une queue de prothèse humérale 1 destinée à être implantée à l'intérieur du canal huméral d'un patient.

Cette queue de prothèse 1 comporte une tige cylindrique 2 et une partie métaphysaire 3 présentant un profil évasé et formant un coude par rapport à la tige cylindrique 2, de façon à présenter une face frontale supérieure 4 de forme circulaire axée sur un axe (α) incliné par rapport à l'axe (β) de ladite tige cylindrique 2.

Cette queue de prothèse 1 comporte également un aileron 5 s'étendant dans le prolongement de la tige cylindrique 2, à l'arrière de la partie métaphysaire 3, et pourvu d'orifices tels que 6 destinés, dans l'hypothèse d'une fracture, à permettre la reconstruction de la partie supérieure de l'humérus autour de la prothèse.

La partie métaphysaire 3 de cette tige cylindrique 2 présente enfin une cavité hémisphérique 7 axée sur l'axe (α) et ménagée à partir de la face frontale supérieure 4, et un alésage taraudé 8, débouchant dans le fond de cette cavité 7 et également axé sur l'axe (α).

La prothèse humérale comporte également une platine humérale 9 destinée à assurer la liaison entre la queue de prothèse 1 et la calotte décrite ci-après.

Cette platine humérale 9 comprend, en premier lieu, une rotule hémisphérique creuse 10 destinée à coopérer avec la portée hémisphérique que forme la cavité 7 de la partie métaphysaire 3 de la queue de prothèse 1, et présentant à cet effet une paroi externe 10a de même rayon que celui de cette cavité 7 au jeu de fonctionnement près. Cette rotule hémisphérique 10 étant creuse, elle présente, en outre, une paroi interne 10b définissant une portée hémisphérique. En dernier lieu, cette rotule hémisphérique 10 est percée d'un alésage 11 de forme tronconique axé sur l'axe de révolution (D) de ladite rotule. Cet alésage 11 présente, en outre, un diamètre croissant de la paroi interne 10a vers la paroi externe 10b de la rotule, et un diamètre minimal supérieur à celui de l'alésage taraudé 8 de la queue de prothèse 1.

La platine humérale 9 comporte également un pivot 12 de forme tronconique surmontant la rotule hémisphérique 10 et relié à la paroi frontale supérieure plane de cette dernière par un tronçon de jonction 13 cylindrique axé sur l'axe de révolution (D) de ladite rotule, de même diamètre interne que le diamètre interne maximal de cette rotule 10, et de diamètre externe sensiblement inférieur au diamètre externe maximal de celle-ci. Ce pivot 12 présente également une paroi supérieure 12a dans laquelle sont ménagées deux trous borgnes tels que 15.

Ce pivot conique 12 présente, enfin, une section moyenne de dimensions supérieures à celles de la section maximale de la rotule hémisphérique 10, dans un rapport de l'ordre de 1,3 à 1,5, et est disposé par rapport à cette dernière de façon que son axe de révolution (D') soit excentré d'une distance d de l'ordre de cinq millimètres par rapport à l'axe de révolution (D) de ladite rotule.

La prothèse humérale comporte, en outre, un organe 16 de blocage relatif de la platine humérale 9 et de la queue de prothèse 1.

Cet organe de blocage 16 comporte, en premier lieu, une tige cylindrique 17 dotée d'un tronçon d'extrémité inférieure 17a fileté adapté pour venir se visser dans l'alésage taraudé 8.

Cette tige 17 est, en outre, surmontée d'une rotule hémisphérique 18 destinée à coopérer avec la portée hémisphérique que forme la face interne 10b de la rotule 10 de la platine humérale 9, et comportant à cet effet une paroi externe, de contact avec cette portée hémisphérique, présentant un même rayon que celui de ladite portée au jeu de fonctionnement près.

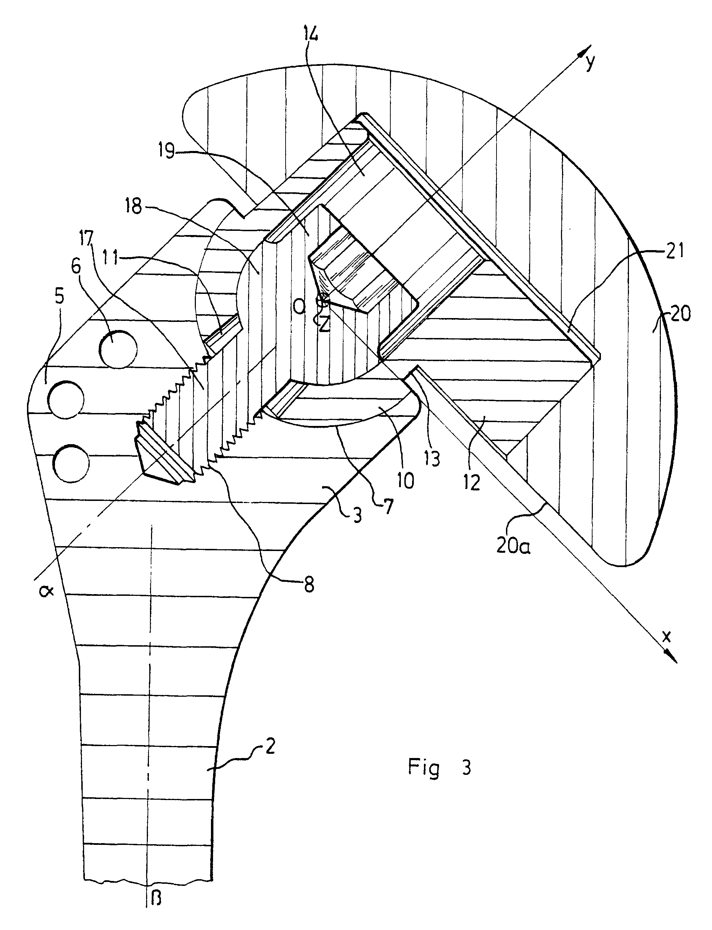

L'organe de blocage 16 comporte enfin une tête de vis 19 coaxiale avec l'axe de révolution de la rotule hémisphérique 18, par exemple du type à six pans, permettant d'assurer le blocage relatif dudit organe de blocage, de la platine humérale 9 et de la queue de prothèse 1, au moyen de tout outil approprié. En outre, tel que représenté à la figure 3, une fois ce blocage effectué, les différentes portées sphériques et rotules sphériques sont concentriques autour d'un même point O.

La prothèse humérale comporte enfin une calotte 20, dite sphérique, destinée à venir coopérer avec la glène, prothétique ou non, de l'épaule, et comportant une paroi inférieure circulaire 20a dans laquelle est ménagée un logement tronconique 21 de forme conjuguée de celle du pivot conique 12, adaptée pour coiffer ce dernier. De plus, l'axe de symétrie (D") de cette calotte 20 est excentré d'une distance de l'ordre de 1,5 mm par rapport à l'axe de symétrie D' du pivot tronconique 12, de sorte que l'excentricité dudit axe (D") par rapport à l'axe de révolution (D) de la rotule hémisphérique 10 peut varier dans une plage de distances comprises entre d - d' et d + d'.

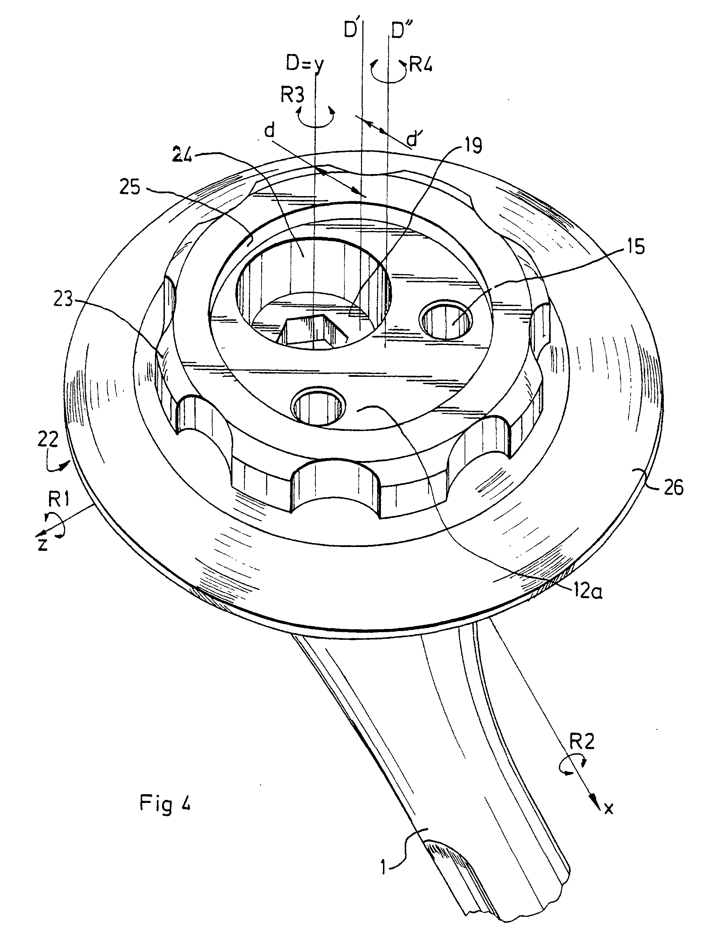

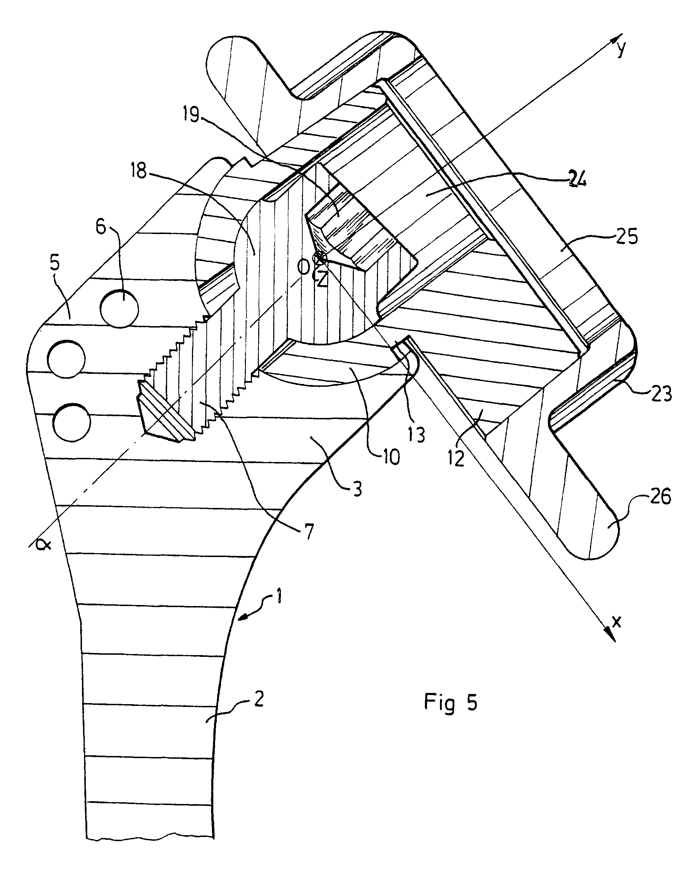

Les calottes d'essai 22 représentées aux figures 4 et 5 sont conçues pour permettre d'effectuer les réglages des paramètres de reconstruction de l'extrémité supérieure de l'humérus, une fois la queue de prothèse 1 implantée et cimentée dans le canal huméral.

Ces calottes d'essai 22 comportent, en premier lieu, une tête cylindrique creuse de préhension 23 moletée, d'axe de révolution D" délimitant un logement tronconique 24, de forme conjuguée de celle du pivot conique 12 adaptée pour loger ce dernier, et dotée d'une face supérieure 25 ouverte en vue de permettre d'accéder à l'organe de blocage 16 une fois lesdites calottes montées sur lesdits pivots.

Ces calottes d'essai 22 comportent, en outre, à la base de la tête de préhension 23, une couronne 26 de même diamètre externe que celui de la calotte définitive 20 correspondante.

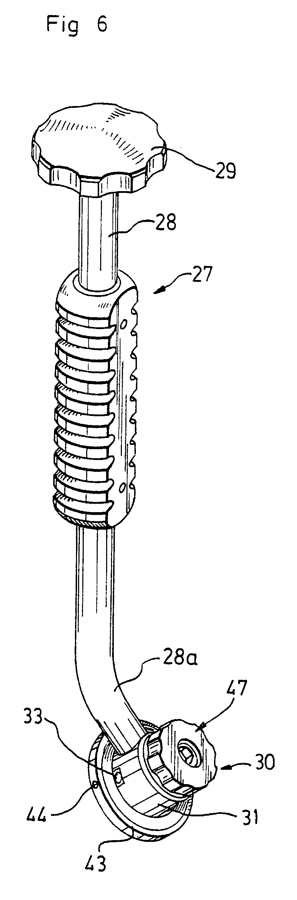

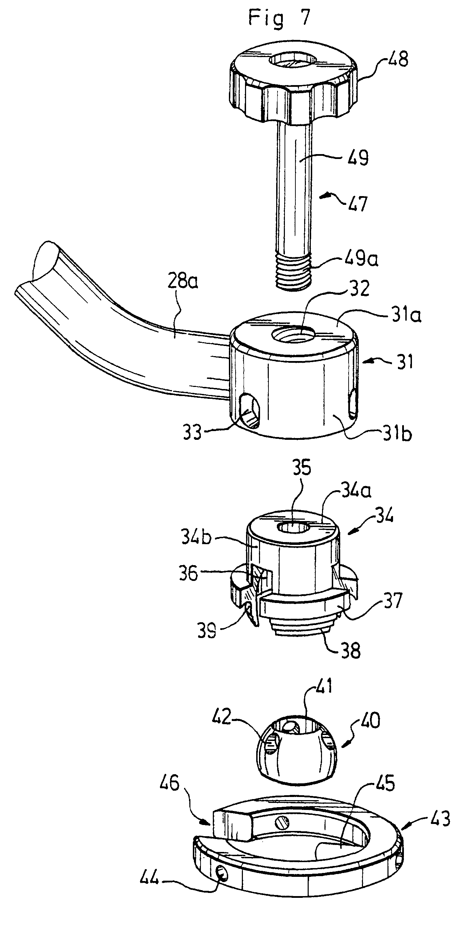

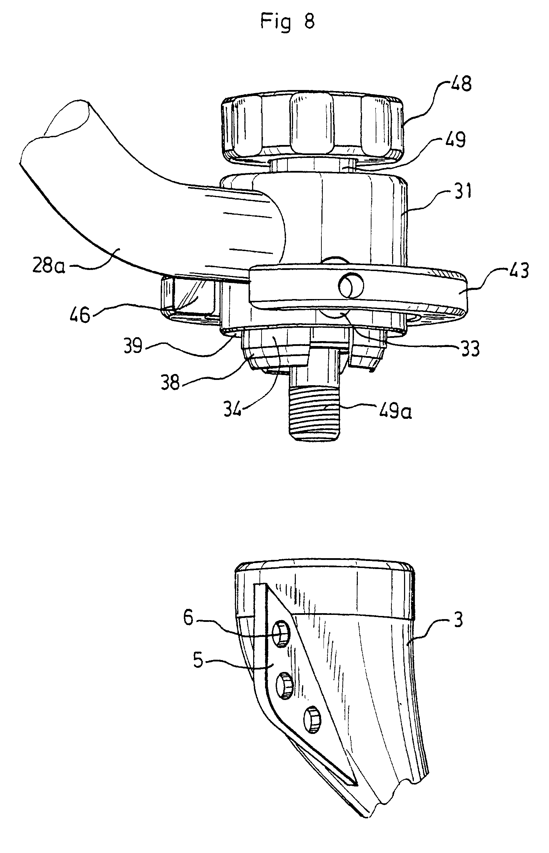

Le préhenseur 27 représenté aux figures 6 à 9 comporte quant à lui un manche cylindrique 28 doté d'une molette supérieure de préhension 29, et présentant un tronçon inférieur 28a coudé d'un angle conjugué de l'angle d'inclinaison de la partie métaphysaire 3 de la queue de prothèse 1.

Ce préhenseur comprend, en outre, une tête 30 de préhension de la partie métaphysaire 3 de la queue de prothèse 1 destinée à coopérer avec cette dernière, après retrait de la platine humérale 9, en vue de l'implanter dans un canal huméral.

Cette tête de préhension 30 comporte, en premier lieu, un capuchon cylindrique 31, en matière métallique, solidaire de l'extrémité inférieure 28a du manche 28, et comportant une paroi supérieure 31a percée axialement d'un orifice circulaire 32, une paroi latérale 31b percée, à l'opposé de la paroi supérieure 31a, de trois orifices oblongs tels que 33 uniformément répartis autour de l'axe dudit boîtier, et une face inférieure ouverte.

Cette tête de préhension 30 comporte également une coiffe interne de forme générale cylindrique 34, en un matériau plastique, de forme adaptée pour être introduite en force dans le capuchon 31. Cette coiffe interne 34 comporte, en premier lieu, une paroi supérieure 34a percée axialement d'un orifice circulaire 35, et une paroi latérale 34b présentant trois échancrures telles que 36 ménagées de façon à se trouver en regard des orifices oblongs 33 du capuchon 31.

Cette coiffe interne 34 comporte, en outre, un rebord périphérique inférieur 37 agencé pour venir en butée contre le bord inférieur du capuchon 31, et présentant des entailles en regard des échancrures 36 dudit capuchon. Elle présente par ailleurs une cavité interne formant une portée hémisphérique prolongée par une jupe inférieure incurvée 38 conférant un caractère rétentif à cette portée hémisphérique. En dernier lieu, cette coiffe interne 34 comporte une gorge annulaire 39 présentant une section en forme de bec adaptée pour coiffer le bord supérieur de la cavité hémisphérique 7 de la queue de prothèse 1, ménagée en sous-face du rebord périphérique 37.

La tête de préhension 30 comporte également une rotule sphérique tronquée 40, en un matériau métallique, de dimensions adaptées pour venir se loger à l'intérieur de la coiffe interne 34. Cette rotule 40 présente en premier lieu un alésage longitudinal 41 de diamètre supérieur à celui des orifices 32, 35 du capuchon 31 et de la coiffe interne 34. Elle présente également trois orifices radiaux tels que 42 uniformément répartis autour de l'axe de symétrie de cette rotule 40, et ménagés de façon à se trouver chacun en regard d'un orifice oblong 33 du capuchon 31.

La tête de préhension 30 comporte, en outre, une couronne 43, en un matériau plastique, de diamètre interne adapté pour être disposé autour du rebord périphérique 37 de la coiffe interne 34. Cette couronne 43 est percée de trois orifices radiaux tels que 44 uniformément répartis, ménagés de façon à permettre de la solidariser à la rotule sphérique 40 au moyen de goupilles métalliques telles que 45 s'étendant au travers des orifices oblongs 33 du capuchon 31 et des échancrures 36 de la coiffe interne 34. Ces goupilles 45 présentent, en outre, un diamètre inférieur aux dimensions des trous oblongs 33, adapté pour autoriser des débattements angulaires de la couronne 43 et de la rotule 40 relativement à la coiffe interne 34 et au capuchon 31. Afin de permettre ces débattements angulaires, la couronne 43 présente une ouverture 46 ménagée de façon à être positionnée en regard de l'extrémité coudée 28a du manche 28.

La tête de préhension 30 comprend enfin une vis 47 à tête moletée 48 dotée d'une tige cylindrique 49 présentant un tronçon inférieur 49a fileté, et de diamètre adapté pour s'étendre au travers des orifices 32, 35 du capuchon 31 et de la coiffe interne 34 et de l'alésage 41 de la rotule 40, et pour venir se visser dans l'alésage taraudé 8 de la queue de prothèse 1.

Un tel préhenseur 27 est adapté pour être assemblé de façon rigide avec la queue de prothèse 1 de façon à permettre l'introduction de cette dernière dans un canal huméral. Il est, en outre, conçu de façon à venir coiffer la cavité hémisphérique 7 de cette queue de prothèse 1, de façon à éviter toute pénétration de ciment à l'intérieur de cette cavité 7.

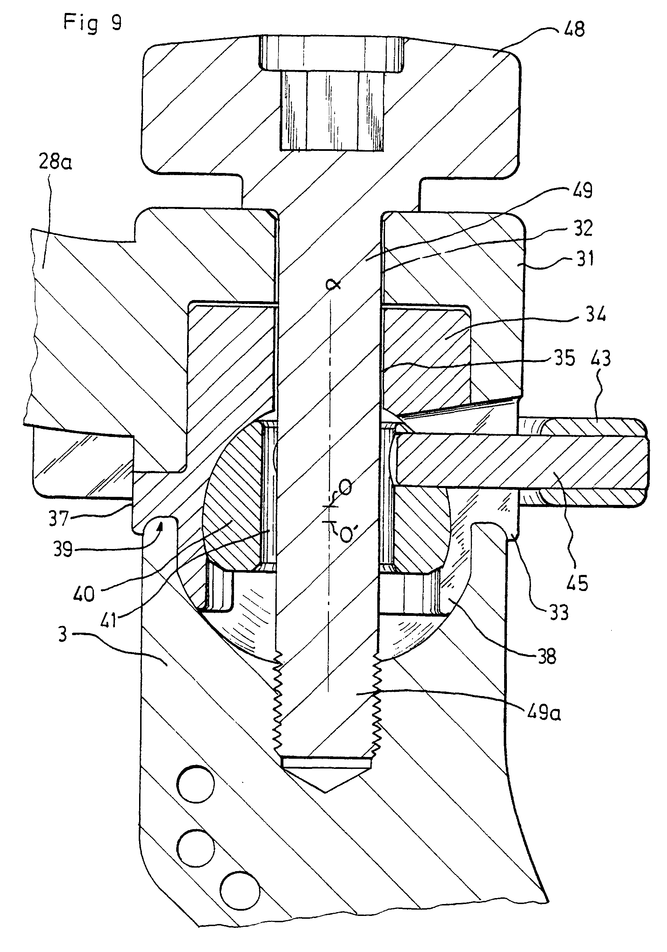

Les caractéristiques dimensionnelles des différents éléments de ce préhenseur sont, en outre, adaptées pour que :
- l'amplitude d'oscillation de la couronne 42, définie par la hauteur des trous oblongs 33, corresponde à l'amplitude des réglages autorisés par la platine humérale 9 décrits en détail ci-après,
- tel que représenté à la figure 9, le centre de rotation O' de la rotule 40 soit décalée vers le bas d'une distance de l'ordre de un mm par rapport au centre O de concentricité des portées hémisphériques et rotules de la prothèse.

Moyennant ces dispositions, un tel préhenseur 27 permet d'implanter la queue de prothèse 1 dans un canal huméral, de façon que la partie métaphysaire 3 de cette dernière soit positionnée de façon optimale garantissant, notamment grâce au décalage des centre O et O', la possibilité ultérieure d'effectuer l'intégralité des réglages autorisés par les liaisons rotulées de la prothèse.

Les différentes étapes d'implantation d'une prothèse au moyen de l'équipement chirurgical ci-dessus décrit sont les suivantes.

En premier lieu, le préhenseur 27 est fixé sur la queue de prothèse 1, et cette dernière est introduite dans le canal de l'humérus, dont la tête a été préalablement coupée de façon classique, jusqu'à ce que la couronne 43 vienne en butée contre la coupe osseuse. A ce stade, la queue de prothèse 1 peut être scellée de façon définitive à l'aide de ciment préalablement introduit dans le canal et dont l'excédent est refoulé.

Le préhenseur 27 est alors retiré, et la platine humérale 9 mise en place par l'intermédiaire de l'organe de blocage 16, ce dernier n'étant pas totalement vissé afin de permettre la rotation de la rotule 10 à l'intérieur de la cavité hémisphérique 7.

Une calotte d'essai 22 de diamètre adéquat est ensuite positionnée sur le pivot conique 12 et l'opérateur peut restituer la géométrie de l'extrémité supérieure de l'humérus grâce aux différents réglages qui lui sont offerts et qui peuvent être décomposés en quatre rotations élémentaires :
- une rotation R1 de centre O autour d'un axe Oz qui permet d'incliner la platine humérale 9 et donc la calotte 22 par rapport à l'axe (β) de la queue de prothèse 1,
- une rotation R2 de centre O autour d'un axe Ox qui permet un réglage de la rétroversion de la calotte 22,
- une rotation R3 de centre O autour d'un axe Oy qui, associée à l'excentration (d) entre les axes D et D' permet un premier réglage du déport combiné,
- et une rotation R4 autour de l'axe D" qui, associée à l'excentration (d') entre les axes D' et D" permet un second réglage du déport combiné indépendant du réglage résultant de la rotation R3.

Il est à noter que les rotations R3 et R4 permettent de faire décrire au centre de la calotte 22 un mouvement de satellite par rapport à l'axe (β) de la queue de prothèse 1 qui conduit à un réglage fin, continu et surtout indépendant des notions de déport médial et de déport postérieur.

L'opérateur possède également la possibilité d'effectuer un réglage en translation le long de l'axe Oy afin éventuellement de restituer la longueur normale de l'humérus. A cet effet, il peut, en effet, être prévu, outre des calottes 20 d'épaisseurs différentes, un jeu de platines humérales 9 présentant des tronçons de jonction 13 de hauteurs différentes.

Une fois le positionnement optimal obtenu, l'opérateur peut alors procéder au blocage de l'organe de blocage 16 accessible au travers de la face ouverte 25 de la calotte d'essai 22, le pivot conique 9 étant préférentiellement maintenu en position au moyen d'un instrument de tout type connu en soi, présentant deux branches agencées pour être introduites dans les trous borgnes 15 du pivot conique 12.

Ce blocage de l'organe de blocage 16 conduit à exercer une pression radiale de la rotule hémisphérique 16 contre la portée hémisphérique 10b de la platine humérale 9, et ainsi à verrouiller dans la position souhaitée la platine humérale 9 relativement à la queue de prothèse 1.

En dernier lieu, la calotte d'essai 22 est retirée, et la calotte définitive 20 mise en place.

Une telle conception d'équipement chirurgical présente en premier lieu l'avantage de permettre d'effectuer les réglages de la position de la calotte 20 directement sur la queue de prothèse 1 définitive sans avoir recours à un implant d'essai tel qu'actuellement nécessaire, et de ce fait, de conduire à une réduction du temps opératoire et à une diminution des risques d'erreurs.

De plus, il offre la possibilité d'effectuer une pluralité de réglages indépendants et continus qui conduisent à une reconstruction très précise de l'anatomie humérale.

## Revendications

1. Equipement chirurgical pour l'implantation d'une prothèse totale d'épaule qui comprend :
- une queue de prothèse d'épaule (1) comportant une tige humérale (2), et une partie métaphysaire (3) coudée par rapport à ladite tige humérale dotée d'une face frontale supérieure (4) inclinée par rapport à la tige humérale (2) dans laquelle est ménagée une cavité hémisphérique (7) axée sur un axe (α), et d'un alésage taraudé (8) débouchant dans le fond de la cavité hémisphérique et axé sur l'axe (α),
- une platine humérale (9) comportant :
. une rotule hémisphérique creuse (10) d'axe de révolution (D), présentant une face externe (10a) adaptée pour coopérer avec la cavité (7) de la queue de prothèse (1) et une face interne (10b) définissant une portée hémisphérique, et étant percée axialement d'un alésage tronconique (11),
. un pivot tronconique (12) d'axe de révolution (D') excentré d'une distance d par rapport à l'axe (D), relié à la rotule (10) par un tronçon de jonction (13) de section au plus égale à la section maximale de ladite rotule, lesdits pivot conique et tronçon de jonction étant percés d'un alésage traversant (14) s'étendant dans le prolongement de la cavité de la rotule (10),
- un organe de blocage (16) comportant disposés axialement:
. une tige (17) dotée d'un tronçon inférieur fileté (17a), et présentant un diamètre conjugué de celui de l'alésage (8) de la queue de prothèse (1) et inférieur au diamétre minimal de l'alésage tronconique (11) de la rotule (10) de la platine humérale,
. une rotule hémisphérique (18) adaptée pour coopérer avec la partie hémisphérique (10b) de la rotule (10) de la platine humérale (9),
. et une tête (19) de vissage dudit organe de blocage,
- les cavités hémisphériques (7) de la queue de prothèse (1), les faces externe (10a) et interne (10b) de la rotule (10) de la platine humérale (9) et la rotule (18) de l'organe de blocage (16) étant concentriques autour d'un point O en position verrouillée de l'organe de blocage (16),
- une calotte (20) comportant une paroi supérieure concave destinée à coopérer avec la glène de l'épaule et une paroi inférieure (20a) présentant un évidement tronconique (21) de forme conjuguée du pivot tronconique (12),
- une calotte, dite d'essai (22), présentant une paroi inférieure de mêmes dimensions que celles de la paroi inférieure (20a) de la calotte (20) présentant un évidement tronconique (24) de forme conjuguée du pivot tronconique (12), et une paroi supérieure (23) percée d'une ouverture débouchant dans ledit évidement.

2. Equipement chirurgical selon la revendication 1, **caractérisé en ce qu'**il comprend un préhenseur (27) doté d'un manche (28) et d'une tête de préhension (30) inclinée par rapport audit manche, ladite tête de préhension étant adaptée pour obturer la cavité (7) de la partie métaphysaire (3) de la queue de prothèse et comportant :
- un organe de blocage (47 - 49) doté d'une tige filetée (49a) apte à être vissée dans l'alésage taraudé (8) de la partie métaphysaire (3) de la queue de prothèse (1),
- une couronne (43) montée autour de ladite tête de préhension avec une faculté d'oscillation autour d'un pivot O' de l'axe (α) situé à une distance du fond de la cavité (7) de la queue de prothèse (1) inférieure à celle séparant le point O dudit fond de cavité.

3. Equipement chirurgical selon la revendication 2, **caractérisé en ce que** la tête de préhension (30) du préhenseur (27) comporte :
- un capuchon creux (31, 34) de forme adaptée pour coiffer la face frontale (4) de la queue de prothèse (1), présentant une cavité interne formant une portée hémisphérique, et comprenant une paroi supérieure (31a) percée axialement d'un orifice (32), et une paroi latérale (31b) percée d'orifices oblongs longitudinaux (33),
- une rotule (40) logée dans la cavité interne du capuchon (31, 34) et percée axialement d'un alésage (41) de diamètre supérieur à celui de l'alésage taraudé (8) de la queue de prothèse,
- et des éléments de liaison (45) de la rotule (40) et de la couronne (43) agencés pour s'étendre au travers des orifices oblongs (33) du capuchon (31, 34) et pour autoriser les débattements angulaires desdites rotule et couronne,
- l'organe de blocage (47, 49) consistant en une vis dotée d'une tige (49) agencée pour s'étendre au travers de l'orifice (32) du capuchon et de l'alésage (41) de la rotule (40).

4. Equipement chirurgical selon l'une des revendications précédentes **caractérisé en ce que** les calottes (20, 22) présentent un axe de révolution D" excentré d'une distance d' par rapport à l'axe de révolution (D') du pivot tronconique (12) de la platine humérale (9).

5. Equipement chirurgical selon l'une des revendications précédentes **caractérisé en ce qu'**il comprend un jeu de platines humérales (9) présentant des tronçons de jonction (13) de hauteurs différentes.

6. Equipement chirurgical selon l'une des revendications précédentes **caractérisé en ce que** les calottes d'essai (22) comportent une tête de préhension moletée (23) délimitant l'évidement tronconique (24) et dotée d'une paroi supérieure ouverte (25), et une couronne circulaire externe (26) s'étendant sur le pourtour de la base de ladite tête moletée.

7. Prothèse totale d'épaule qui comprend en combinaison :
- une queue de prothèse d'épaule (1) comportant une tige humérale (2), et une partie métaphysaire (3) coudée par rapport à ladite tige humérale dotée d'une face frontale supérieure (4) inclinée par rapport à la tige humérale (2) dans laquelle est ménagée une cavité hémisphérique (7) axée sur un axe (α), et d'un alésage taraudé (8) débouchant dans le fond de la cavité hémisphérique et axé sur l'axe (α),
- une platine humérale (9) comportant :
. une rotule hémisphérique creuse (10) d'axe de révolution (D), présentant une face externe (10a) adaptée pour coopérer avec la cavité (7) de la queue de prothèse (1) et une face interne (10b) définissant une portée hémisphérique, et étant percée axialement d'un alésage tronconique (11),
. un pivot tronconique (12) d'axe de révolution (D') excentré d'une distance d par rapport à l'axe (D), relié à la rotule (10) par un tronçon de jonction (13) de section au plus égale à la section maximale de ladite rotule, lesdits pivot conique et tronçon de jonction étant percés d'un alésage traversant (14) s'étendant dans le prolongement de la cavité de la rotule (10),
- un organe de blocage (16) comportant disposés axialement :
. une tige (17) dotée d'un tronçon inférieur fileté (17a), et présentant un diamètre conjugué de celui de l'alésage (8) de la queue de prothèse (1) et inférieur au diamètre minimal de l'alésage tronconique (11) de la rotule (10) de la platine humérale,
. une rotule hémisphérique (18) adaptée pour coopérer avec la partie hémisphérique (10b) de la rotule (10) de la platine humérale (9),
. et une tête (19) de vissage dudit organe de blocage,
- les cavités hémisphériques (7) de la queue de prothèse (1), les faces externe (10a) et interne (10b) de la rotule (10) de la platine humérale (9) et la rotule (18) de l'organe de blocage (16) étant concentriques autour d'un point O en position verrouillée de l'organe de blocage (16),
- une calotte (20) comportant une paroi supérieure concave destinée à coopérer avec la glène de l'épaule et une paroi inférieure (20a) présentant un évidement tronconique (21) de forme conjuguée du pivot tronconique (12).

8. Prothèse totale d'épaule selon la revendication 7 **caractérisée en ce que** la calotte (20) présente un axe de révolution D" excentré d'une distance d' par rapport à l'axe de révolution D'du pivot tronconique (12) de la platine humérale (9).

## Patentansprüche

1. Chirurgische Vorrichtung für die Implantation einer Schulter-Totalprothese, die Folgendes umfasst:
- einen Schulterprotheseschaft (1), umfassend einen Oberarmstab (2) und einen Metaphyseteil (3), der in Bezug auf den genannten Oberarmstab abgewinkelt ist, versehen mit einer oberen Frontfläche (4), die in Bezug auf den Oberarmstab (2) geneigt ist und in der ein auf eine Achse (α) bezogener halbkugelförmiger Hohlraum (7) ausgebildet ist, und mit einer Innengewindebohrung (8), die in den Boden des halbkugelförmigen Hohlraums mündet und auf die Achse (α) bezogen ist,
- eine Oberarmplatte (9), die Folgendes umfasst:
. einen hohlen, halbkugelförmigen Drehgelenkkopf (10) mit einer Drehachse (D), der eine Außenfläche (10a) aufweist, die so gestaltet ist, dass sie mit dem Hohlraum (7) des Protheseschafts (1) zusammenwirkt, und eine Innenfläche (10b), die ein halbkugelförmiges Widerlager definiert und axial von einer kegelstumpfförmigen Bohrung (11) durchbohrt ist,
. ein kegelstumpfförmiges Drehgelenk (12) mit einer Drehachse (D'), das um einen Abstand d in Bezug auf die Achse (D) exzentrisch und mit dem Drehgelenkkopf (10) durch ein Übergangsstück (13) verbunden ist, dessen Querschnitt höchstens gleich dem maximalen Querschnitt des genannten Drehgelenkkopfes ist, wobei das genannte konische Drehgelenk und das genannte Übergangsstück von einer Querbohrung (14) durchbohrt sind, die durch die Verlängerung des Hohlraums des Drehgelenkkopfes (10) verläuft,
- einen Sperrmechanismus (16), der in axialer Anordnung Folgendes umfasst:
. einen Stab (17), der einen unteren Außengewindeabschnitt (17a) aufweist und einen Durchmesser hat, der zu dem der Bohrung (8) des Protheseschafts (1) passt und kleiner ist als der minimale Durchmesser der kegelstumpfförmigen Bohrung (11) des Drehgelenkkopfes (10) der Oberarmplatte,
. einen halbkugelförmigen Drehgelenkkopf (18), der so gestaltet ist, dass er mit dem halbkugelförmigen Teil (10b) des Drehgelenkkopfes (10) der Oberarmplatte (9) zusammenwirkt,
. und einen Kopf (19) zum Schrauben des genannten Sperrmechanismus,
- wobei die halbkugelförmigen Hohlräume (7) des Protheseschafts (1), die Außenfläche (10a) und die Innenfläche (10b) des Drehgelenkkopfes (10) der Oberarmplatte (9) und der Drehgelenkkopf (18) des Sperrmechanismus (16) um einen Punkt O in Sperrposition des Sperrmechanismus (16) konzentrisch sind,
- eine Kuppe (20), die eine obere konkave Wand zum Zusammenwirken mit der Pfanne der Schulter und eine untere Wand (20a) aufweist, die eine kegelstumpfförmige Aussparung (21) in einer Form aufweist, die zu dem kegelstumpfförmigen Drehgelenk (12) passt,
- eine Versuchskuppe (22), die eine untere Wand mit denselben Abmessungen wie die der unteren Wand (20a) der Kuppe (20) aufweist, die eine kegelstumpfförmige Aussparung (24) mit einer Form aufweist, die zu der des kegelstumpfförmigen Drehgelenks (12) passt, und eine obere Wand (23), die von einer Öffnung durchbohrt ist, die in die genannte Aussparung mündet.

2. Chirurgische Vorrichtung nach Anspruch 1, **dadurch gekennzeichnet, dass** sie einen Griffteil (27) mit einer Griffstange (28) und einem Griffkopf (30) umfasst, der in Bezug auf die genannte Griffstange geneigt ist, wobei der genannte Griffkopf so gestaltet ist, dass er den Hohlraum (7) des Metaphyseteils (3) des Protheseschafts verschließt und Folgendes umfasst:
- einen Sperrmechanismus (47 - 49), der mit einem Außengewindestab (49a) versehen ist, der in eine Innengewindebohrung (8) des Metaphyseteils (3) des Protheseschafts (1) geschraubt werden kann,
- einen Kranz (43), der um den genannten Griffkopf montiert ist und sich um einen Drehpunkt O' der Achse (α) hin- und herbewegen kann, der sich in einem Abstand vom Boden des Hohlraums (7) des Protheseschafts (1) befindet, der kleiner ist als der, der den Punkt O von dem genannten Boden des Hohlraums trennt.

3. Chirurgische Vorrichtung nach Anspruch 2, **dadurch gekennzeichnet, dass** der Griffkopf (30) des Griffteils (27) Folgendes umfasst:
- eine hohle Kappe (31, 34), die so gestaltet ist, dass sie die Frontfläche (4) des Protheseschafts (1) bedeckt, mit einem inneren Hohlraum, der ein halbkugelförmiges Widerlager bildet, und mit einer oberen Wand (31a), die axial von einem Loch (32) durchbohrt ist, und einer Seitenwand (31b), die von länglichen Längslöchern (33) durchbohrt ist,
- einen Drehgelenkkopf (40), der im inneren Hohlraum der Kappe (31, 34) aufgenommen wird und axial von einer Bohrung (41) mit einem Durchmesser durchbohrt ist, der größer ist als der der Innengewindebohrung (8) des Protheseschafts,
- und Elemente (45) zum Verbinden des Drehgelenkkopfes (40) mit dem Kranz (43), die durch die länglichen Löcher (33) der Kappe (31, 34) verlaufen und die winkelmäßigen Ausfederungen des genannten Drehgelenkkopfes und des genannten Kranzes zulassen,
- den Sperrmechanismus (47, 49), der aus einer Schraube besteht, die mit einem Stab (49) versehen ist, der durch das Loch (32) der Kappe und der Bohrung (41) des Drehgelenkkopfes (40) verläuft.

4. Chirurgische Vorrichtung nach einem der vorherigen Ansprüche, **dadurch gekennzeichnet, dass** die Kuppen (20, 22) eine Drehachse D'' aufweisen, die um einen Abstand d' in Bezug auf die Drehachse (D') des kegelstumpfförmigen Drehgelenks (12) der Oberarmplatte (9) exzentrisch sind.

5. Chirurgische Vorrichtung nach einem der vorherigen Ansprüche, **dadurch gekennzeichnet, dass** sie einen Satz von Oberarmplatten (9) umfasst, die Übergangsabschnitte (13) mit verschiedenen Höhen aufweisen.

6. Chirurgische Vorrichtung nach einem der vorherigen Ansprüche, **dadurch gekennzeichnet, dass** die Versuchskuppen (22) einen gerändelten Griffkopf (23) umfassen, der die kegelstumpfförmige Aussparung (24) begrenzt und mit einer offenen oberen Wand (25) und einem kreisförmigen Außenkranz (26) versehen ist, der auf dem äußeren Umfang der Basis des genannten gerändelten Kopfes verläuft.

7. Schulter-Totalprothese, die in Kombination Folgendes umfasst :
- einen Schulterprotheseschaft (1), umfassend einen Oberarmstab (2) und einen Metaphyseteil (3), der in Bezug auf den genannten Oberarmstab abgewinkelt ist, versehen mit einer oberen Frontfläche (4), die in Bezug auf den Oberarmstab (2) geneigt ist und in der ein auf eine Achse (α) bezogener halbkugelförmiger Hohlraum (7) ausgebildet ist, und mit einer Innengewindebohrung (8), die in den Boden des halbkugelförmigen Hohlraums mündet und auf die Achse (α) bezogen ist,
- eine Oberarmplatte (9), die Folgendes umfasst:
. einen hohlen, halbkugelförmigen Drehgelenkkopf (10) mit einer Drehachse (D), der eine Außenfläche (10a) aufweist, die so gestaltet ist, dass sie mit dem Hohlraum (7) des Protheseschafts (1) zusammenwirkt, und eine Innenfläche (10b), die ein halbkugelförmiges Widerlager definiert und axial von einer kegelstumpfförmigen Bohrung (11) durchbohrt ist,
. ein kegelstumpfförmiges Drehgelenk (12) mit einer Drehachse (D'), das um einen Abstand d in Bezug auf die Achse (D) exzentrisch und mit dem Drehgelenkkopf (10) durch ein Übergangsstück (13) verbunden ist, dessen Querschnitt höchstens gleich dem maximalen Querschnitt des genannten Drehgelenkkopfes ist, wobei das genannte konische Drehgelenk und das genannte Übergangsstück von einer Querbohrung (14) durchbohrt sind, die durch die Verlängerung des Hohlraums des Drehgelenkkopfes (10) verläuft,
- einen Sperrmechanismus (16), der in axialer Anordnung Folgendes umfasst:
. einen Stab (17), der einen unteren Außengewindeabschnitt (17a) aufweist und einen Durchmesser hat, der zu dem der Bohrung (8) des Protheseschafts (1) passt und kleiner ist als der minimale Durchmesser der kegelstumpfförmigen Bohrung (11) des Drehgelenkkopfes (10) der Oberarmplatte,
. einen halbkugelförmigen Drehgelenkkopf (18), der so gestaltet ist, dass er mit dem halbkugelförmigen Teil (10b) des Drehgelenkkopfes (10) der Oberarmplatte (9) zusammenwirkt,
. und einen Kopf (19) zum Schrauben des genannten Sperrmechanismus,
- wobei die halbkugelförmigen Hohlräume (7) des Protheseschafts (1), die Außenfläche (10a) und die Innenfläche (10b) des Drehgelenkkopfes (10) der Oberarmplatte (9) und der Drehgelenkkopf (18) des Sperrmechanismus (16) um einen Punkt O in Sperrposition des Sperrmechanismus (16) konzentrisch sind,
- eine Kuppe (20), die eine obere konkave Wand zum Zusammenwirken mit der Pfanne der Schulter und eine untere Wand (20a) aufweist, die eine kegelstumpfförmige Aussparung (21) in einer Form aufweist, die zu dem kegelstumpfförmigen Drehgelenk (12) passt .

8. Schulter-Totalprothese nach Anspruch 7, **dadurch gekennzeichnet, dass** die Kuppe (20) eine Drehachse D'' aufweist, die um einen Abstand d' in Bezug auf die Drehachse D' des kegelförmigen Drehgelenks (12) der Oberarmplatte (9) exzentrisch ist.

## Claims

1. Surgical equipment for implantation of a full shoulder prosthesis comprising:
- a shoulder prosthesis tail (1) comprising a humeral rod (2) and a metaphyseal part (3) which is elbowed relative to the said humeral rod and is provided with an upper front surface (4) which is inclined relative to the humeral rod (2) in which there is provided a hemispherical cavity (7) which is centred on an axis (α), and with a threaded bore (8) which opens into the base of the hemispherical cavity and is centred on the axis (α);
- a humeral plate (9) comprising:
. a hollow hemispherical ball joint (10) with an axis of revolution (D), with an outer surface (10a) which is designed to co-operate with the cavity (7) of the tail (1) of the prosthesis and an inner surface (10b) which defines a hemispherical support surface, and is provided with a frusto-conical axial bore (11);
- a frusto-conical pivot (12) with an axis of revolution (D') which is off-centred by a distance d relative to the axis (D), is connected to the ball joint (10) by a joining section (13) with a cross-section which is at the most equal to the maximum cross-section of the said ball joint, the said conical pivot and joining section being provided with a through bore (14) which extends in the extension of the cavity of the ball joint (10);
- a locking unit (16) comprising the following disposed axially:
. a rod (17) provided with a threaded lower section (17a) and having a diameter which is conjugated with that of the bore (8) in the tail (1) of the prosthesis and is smaller than the minimum diameter of the frusto-conical bore (11) in the ball joint (10) of the humeral plate;
- a hemispherical ball joint (18) which is designed to co-operate with the hemispherical part (10b) of the ball joint (10) of the humeral plate (9); and
. a screwing head (19) of the said locking unit;
- the hemispherical cavities (7) of the tail (1) of the prosthesis, the outer (10a) and inner (10b) surfaces of the ball joint (10) of the humeral plate (9) and the ball joint (18) of the locking unit (16) being concentric around a point O in the locked position of the locking unit (16);
- a cap (20) comprising a concave upper wall which is designed to co-operate with the shoulder socket and a lower wall (20a) which has a frusto-conical recess (21) with a shape which is conjugated with that of the frusto-conical pivot (12); and
- a cap, known as the test cap (22) having a lower wall with the same dimensions as those of the lower wall (20a) of the cap (20) having a frusto-conical recess (24) with a shape which is conjugated with the frusto-conical pivot (12), and an upper wall (23) provided with an opening which opens into the said recess.

2. Surgical equipment according to claim 1, **characterised in that** it comprises a gripping unit (27) provided with a sleeve (28) and with a gripping head (30) which is inclined relative to the said sleeve, the said gripping head being designed to close the cavity (7) of the metaphyseal part (3) of the tail of the prosthesis and comprising:
- a locking unit (47 - 49) provided with a threaded rod (49a) which can be screwed into the threaded bore (8) in the metaphyseal part (3) of the tail (1) of the prosthesis; and
- a ring (43) which is fitted around the said gripping head and can oscillate around a pivot O' of the axis (α) situated at a distance from the base of the cavity (7) of the tail (1) of the prosthesis which is less than that which separates the point O from the said base of the cavity.

3. Surgical equipment according to claim 2, **characterised in that** the gripping head (30) of the gripping unit (27) comprises:
- a hollow cap (31, 34) with a shape which is designed to cover the front surface (4) of the tail (1) of the prosthesis, with an inner cavity which forms a hemispherical support surface, and comprising an upper wall (31a) which contains an axial aperture (32), and a lateral wall (31b) which contains longitudinal oblong apertures (33);
- a ball joint (40) which is accommodated in the inner cavity of the cap (31, 34) and contains an axial bore (41) with a diameter larger than that of the threaded bore (8) of the tail of the prosthesis; and
- elements (45) for connection of the ball joint (40) and of the ring (43) which are designed to extend through oblong apertures (33) in the cap (31, 34) and to permit angular clearance of the said ball joint and ring;
- the locking unit (47, 49) consisting of a screw which is provided with a rod (49) designed to extend through the aperture (32) in the cap and the bore (41) in the ball joint (40).

4. Surgical equipment according to any one of the preceding claims, **characterised in that** the caps (20, 22) have an axis of revolution D" which is off-centred by a distance d' relative to the axis of revolution (D') of the frusto-conical pivot (12) of the humeral plate (9).

5. Surgical equipment according to any one of the preceding claims, **characterised in that** it comprises a set of humeral plates (9) which have joining sections (13) with different heights.

6. Surgical equipment according to any one of the preceding claims, **characterised in that** the test caps (22) comprise a knurled gripping head (23) which delimits the frusto-conical recess (24) and is provided with an open upper wall (25), and an outer circular ring (26) which extends around the periphery of the base of the said knurled head.

7. Full shoulder prosthesis which comprises in combination:
- a shoulder prosthesis tail (1) comprising a humeral rod (2), and a metaphyseal part (3) which is elbowed relative to the said humeral rod and is provided with an upper front surface (4) which is inclined relative to the humeral rod (2) in which there is provided a hemispherical cavity (7) which is centred on an axis (α), and with a threaded bore (8) which opens into the base of the hemispherical cavity and is centred on the axis (α);
- a humeral plate (9) comprising:
. a hollow hemispherical ball joint (10) with an axis of revolution (D), with an outer surface (10a) which is designed to co-operate with the cavity (7) of the tail (1) of the prosthesis and an inner surface (10b) which defines a hemispherical support surface, and is provided with a frusto-conical axial bore (11);
- a frusto-conical pivot (12) with an axis of revolution (D') which is off-centred by a distance d relative to the axis (D), is connected to the ball joint (10) by a joining section (13) with a cross-section which is at the most equal to the maximum cross-section of the said ball joint, the said conical pivot and joining section being provided with a through bore (14) which extends in the extension of the cavity of the ball joint (10);
- a locking unit (16) comprising the following disposed axially:
a rod (17) provided with a threaded lower section (17a) and having a diameter which is conjugated with that of the bore (8) in the tail (1) of the prosthesis and is smaller than the minimum diameter of the frusto-conical bore (11) in the ball joint (10) of the humeral plate;
- a hemispherical ball joint (18) which is designed to co-operate with the hemispherical part (10b) of the ball joint (10) of the humeral plate (9); and
. a screwing head (19) of the said locking unit;
- the hemispherical cavities (7) of the tail (1) of the prosthesis, the outer (10a) and inner (10b) surfaces of the ball joint (10) of the humeral plate (9) and the ball joint (18) of the locking unit (16) being concentric around a point O in the locked position of the locking unit (16); and
- a cap (20) comprising a concave upper wall which is designed to co-operate with the shoulder socket and a lower wall (20a) which has a frusto-conical recess (21) with a shape which is conjugated with that of the frusto-conical pivot (12).

8. Full shoulder prosthesis according to claim 7, **characterised in that** the cap (20) has an axis of revolution D" which is off-centred by a distance d' relative to the axis of revolution D' of the frusto-conical pivot (12) of the humeral plate (9).
